# EUROPEAN PATENT APPLICATION

(11) **EP 1 108 372 A1**
(43) Date of publication of application: **20.06.2001**
(21) Application number: 99959733.9
(22) Date of filing: 09.12.1999
(51) Int. Cl.: A41B 9/02

(54) **METHOD OF PRODUCING TRUNKS TYPE WEARING ARTICLES**

(30) Priority: 10.12.1998 JP 35184398
(71) Applicant: UNI-CHARM CO., LTD., Kawanoe-shi Ehime 799-0111 (JP)
(72) Inventor: MURAKAMI, Masaki UNI-Charm Co., Ltd., Mitoyo-gun Kawaga 769-1602 (JP); MATSUSHITA, Michiyo UNI-Charm Co., Ltd., Mitoyo-gun Kagawa 769-1602 (JP)
(74) Representative: Murgatroyd, Susan Elizabeth
(86) International application number: JP9906924
(87) International publication number: WO0033681

(57) **Abstract**

In a process for making a trunks-type garment according to this invention, middle bodies 6, 7 defining a crotch region of trunks-type garment 1 are made from first and second sub-sheets obtained by cutting a main web continuously fed in one direction along a sinusoidal wave-like line extending longitudinally of the main web, and the first and second sub-sheets are bonded to each other along crests of these two sub-sheets described by the sinusoidal wave-like curve to obtain a laminated sheet which is then cut transversely thereof.

## Description

### FIELD OF THE INVENTION

This invention relates to a process for a making trunks-type garment adapted to be used as an upper garment or an undergarment.

### BACKGROUND ART

It is well known to form a crotch region of trunks-type garment by putting a pair of sheets each having a U-shaped cutout flat together with the cutouts of these two sheets being aligned with each other and bonding these two sheets along peripheries of the cutouts.

The process according to the prior art as has been mentioned above necessarily generates U-shaped cuttings of the sheets as they are formed with the cutouts. These cuttings of the sheets will significantly affect a manufacturing cost of the garment so far as the cuttings of the sheets are not directly utilized to make the trunks even if these cuttings of the sheets are not scrapped as they are.

This invention aims to use sheet material for a crotch region of trunks-type garment without generation of waste cuttings of the sheet material and thereby to reduce a manufacturing cost of this article.

### SUMMARY OF THE INVENTION

The object set forth above is achieved, according to this invention, by a process for making trunks-type garment having a pair of lateral bodies intended to cover a wearer's both sides and a middle body intended to cover middle and crotch regions of the wearer's trunk and bonded to the lateral bodies on belly and back sides of the wearer, the process being characterized by that the middle body is made at least through the steps of:
a. cutting a main web having first and second side sections extending in parallel to each other and continuously fed in one direction along a first sinusoidal wave-like curve extending in the one direction so as to divide the main web in two, the first curve having a given amplitude and defined by crests and troughs alternating every 1/2 wavelength, to obtain a first web having the first side section and a third side section extending substantially in parallel to the first side section so as to be contoured by the first sinusoidal wave-like curve and a second web having the second side section and a fourth side section contoured by the first sinusoidal wave-like curve from the main web;
b. bonding one of the second web and third web having a rectilinear fifth side section and a sixth side section extending substantially in parallel to the fifth side section so as to be contoured by a second sinusoidal curve having the same wavelength as the first sinusoidal curve to the first web so that the crests as well as the troughs of the one web face the corresponding crests as well as the corresponding troughs of the first web, respectively, and the corresponding crests of these two webs partially overlap in the vicinity of their summits, respectively, to obtain an apertured sheet of which each aperture is formed by a pair of the troughs facing each other;
c. placing a pair of the apertured sheets one upon another so that the apertures of these two sheets are exactly aligned with each other and bonding these two sheets along peripheries of the apertures to obtain a laminated sheet; and
d. cutting the laminated sheet along a first cutting line extending transversely of the laminated sheet so as to divide the aperture in two and along a second cutting line extending transversely of the laminated sheet so as to divide a section defined between a pair of adjacent apertures in two to obtain the middle body.

According to one preferred embodiment of this invention, the step b) is preceded by a step of dividing a second main web continuously fed in one direction along the second sinusoidal wave-like curve in two sections and obtaining the third web from these two sections.

According to another preferred embodiment of this invention, the first and second webs are made of a nonwoven fabric and/or woven fabric having external appearances and/or physical attributes differing from those of a nonwoven fabric or a woven fabric used as stock material for the third web.

According to still another preferred embodiment of this invention, the first and second sinusoidal wave-like curves are identical in the wavelength but different in the waveform.

### BRIEF DESCRIPTION OF THE DRAWINGS

Fig. 1 is a perspective view showing partially cutaway trunks obtained by the process according to this invention;
Fig. 2 is a plan view showing a partially cutaway middle body;
Fig. 3 is a schematic diagram illustrating steps of the process for making the trunks according to one preferred embodiment of this invention; and
Fig. 4 is a view similar to Fig. 3 illustrating the steps of the process for making the trunks according to another preferred embodiment of this invention.

### DESCRIPTION OF THE BEST MODE FOR WORKING OF THE INVENTION

Details of a process in accordance with this invention to make a trunks-type garment will be more fully understood from the description given hereunder by way of an example with reference to the accompanying drawings which illustrate the case of disposable trunks-type garment.

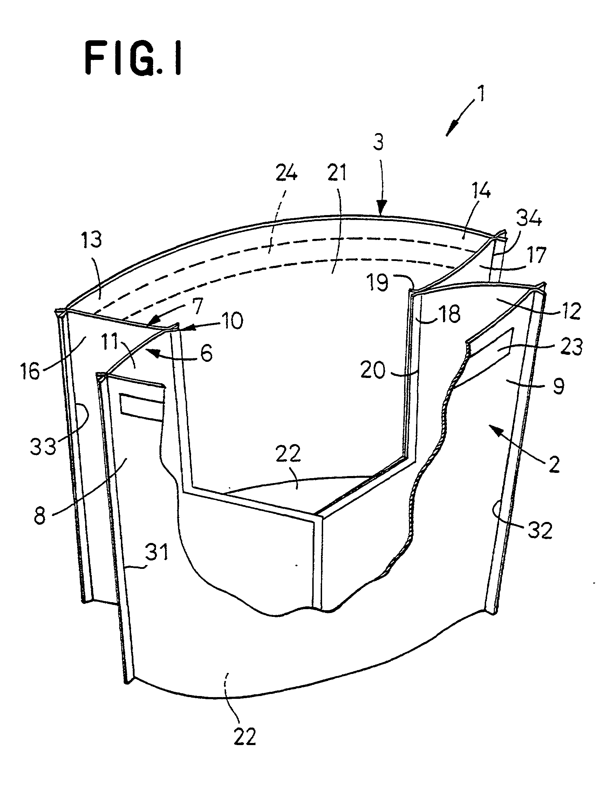

Partially cutaway disposable trunks 1 shown by Fig. 1 in a perspective view generally comprise left- and right-hand lateral bodies 2, 3 intended to cover left- and right-hand lateral regions of a wearer's trunk, respectively, and left- and right-half middle bodies 6, 7 defining a crotch region 10 substantially intended to cover front, rear and crotch regions of the wearer's trunk. It should be understood here that the portion of the trunks 1 on the left-hand as viewed in Fig. 1 is intended to be placed in front of the wearer's trunk. The left-hand lateral body 2 has front and rear side portions 8, 9 which are bonded to front and rear side portions 11, 12 of the left-half middle body 6 along sealing lines 31, 32, respectively. Similarly, the right-hand lateral body 3 has front and rear side portions 13, 14 which are bonded to front and rear side portions 16, 17 of the right-half middle body 7 along sealing lines 33, 34, respectively. The left- and right half middle bodies 6, 7 are respectively formed with U-shaped cutouts 18, 19 defining transitions between the front and rear side portions of these middle bodies 6, 7. The left- and right-half middle bodies 6, 7 are bonded to each other along a sealing line 20 defined by edges of the cutouts 18, 19. The respective bodies 2, 3, 6, 7 are bonded one to another so as to form a waist-opening 21 and a pair of leg-openings 22, 22 of the trunks 1. The left- and right-hand lateral bodies 2, 3 are respectively provided along the associated edges of the waist-opening 21 with elastic members 23, 24 bonded under tension to these bodies 2, 3 so that the trunks 1 may be elastically stretchable and contractible in its circumferential direction.

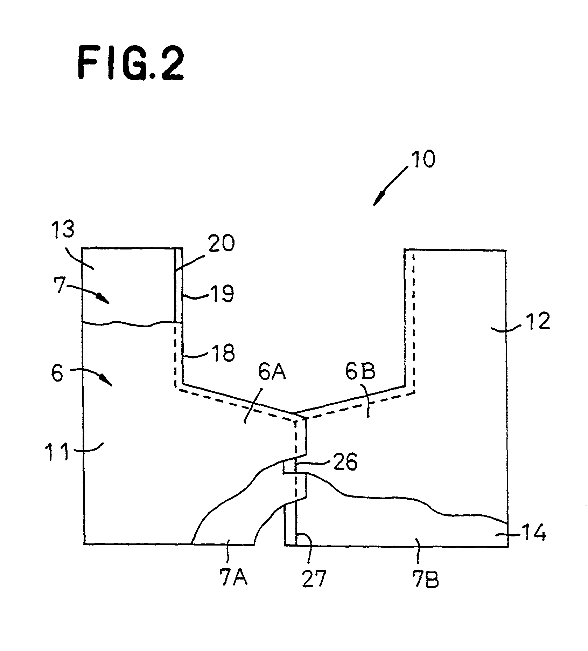

Fig. 2 is a plan view of the crotch member 10 obtained by peeling the left- and right-hand lateral bodies 2, 3 off from the left- and right-half middle bodies 6, 7. Of the left- and right-half middle bodies 6, 7 which are identical to each other in the size as well as in the shape, the left-half middle body 6 comprises front and rear bodies 6A, 6B bonded to each other along a sealing line 26 and, similarly, the right-half middle body 7 comprises front and rear bodies 7A, 7B bonded to each other along a sealing line 27.

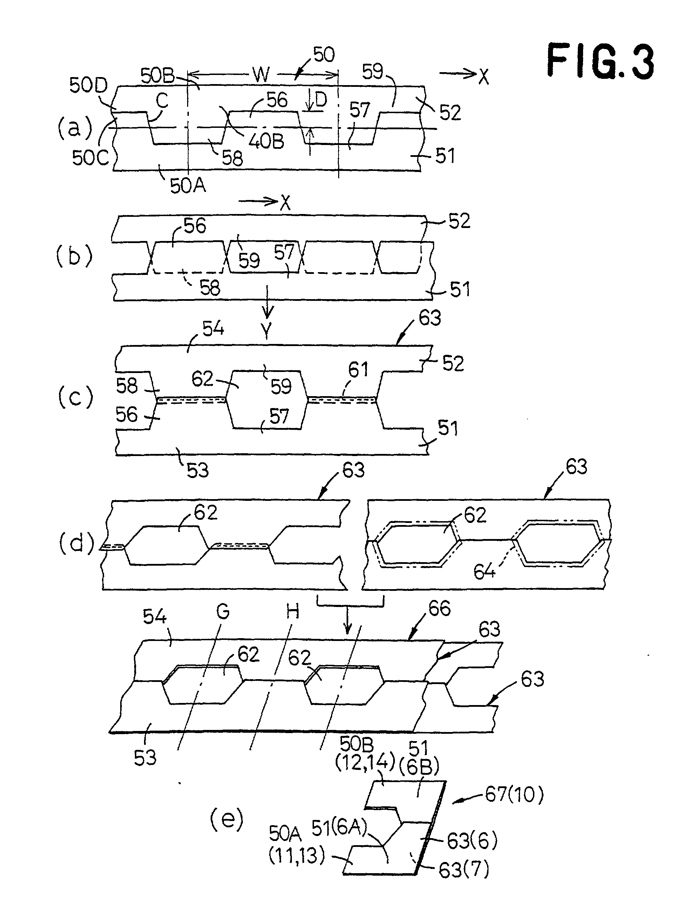

Fig. 3 is a diagram illustrating the steps of the process for making the left-half middle body 6 in a continuous manner.

The process according to this invention will be described step by step first in reference with Fig. 3. On a step a), a main web 50 having first and second side sections 50A, 50B extending in parallel to each other as illustrated is continuously fed in a direction indicated by an arrow X. The main web 50 being thus continuously fed is cut transversely in two along a sinusoidal wave-like curve C of given wavelength W and amplitude D describing crests and troughs alternating every 1/2 wavelength to obtain first and second webs 51, 52. The first web 51 has the first side section 50A and a third side section 50C extending substantially in parallel to the first side section 50A so as to be contoured by crests 56 and troughs 57. The second web 52 has the second side section 50B and a fourth side section 50D contoured by crests 58 and troughs 59.

On a step b), one of the first and second webs 51, 52 may be shifted by 1/2 wavelength in the direction as indicated by the arrow X or in the direction opposed thereto so that the crests 56, 58 as well as the troughs 57, 59 of the first and second webs 51, 52 may face each other, respectively.

On a step c), one of the first and second webs 51, 52 may be shifted in a direction as indicated by an arrow Y (See Fig. 3(c) )or in a direction opposed thereto so far as the crests 56, 58 remain in their mutually overlapping relationship. The crests 56, 58 thus mutually overlapping may be bonded together along sealing lines 61 to obtain an apertured sheet 63 having apertures 62 defined by the troughs 57, 59 of the first and second webs 51, 52, respectively.

On a step d), a pair of the apertured sheets 63, 63 are laminated with each other so that the apertures 62 of the one sheet 63 are aligned with the corresponding apertures 62 of the other sheet 63, respectively, and bonded to each other by a sealing lines 64 extending along periphery of the apertures 62. In this way, a laminated sheet 66 is obtained. Fig. (3d) illustrate these two apertured sheets 63, 63 in a perspective view in order to clarify the manner in which they are laminated with each other.

On a step e), the laminated sheet 66 is cut along a first cutting line G (See Fig. 3 (d)) extending transversely of the sheet 66 so as to divide the aperture 62 in two and along a second cutting line H (See Fig. 3 (d)) also extending transversely of the sheet 66 so as to divide a section of the sheet 66 defined between a pair of adjacent apertures 62 in two. In this manner, a crotch member unit 67 is obtained.

The unit 67 obtained by the step e) corresponds to the crotch member 10 of Fig. 2. Specifically, the pair of apertured sheets 63, 63 laminated with each other are destined to define the left- and right-half middle bodies 6, 7, respectively, of the crotch member 10. The first and second webs 51, 52 are destined to define the front bodies 6A, 7A and the rear bodies 6B, 7B of the middle bodies 6, 7, respectively. The sealing line 61 of the apertured sheet 63 is intended to define the sealing lines 26, 27 in Fig. 2 and the sealing line 64 extending along the periphery of the aperture 62 is intended to define the sealing line 20 in Fig. 2. The first side section 50A of the main web 50 is destined to define the side sections 11, 13 and the second side section 50B of the main web 50 is intended to define the side sections 12, 14 in Fig. 2.

The unit 67 comprises the apertured sheets 63, 63 and the sheets destined to define the left- and right-hand lateral bodies 2, 3 of the trunks 1, which are placed upon the respective outer surfaces of the apertured sheets 63, 63 and bonded thereto at the first and second side sections 50A, 50B of the respective apertured sheets 63, 63. The side sections 2, 3 are provided on their outer surfaces with the elastic members 23, 24 bonded under tension thereto. It is also possible to bond these elastic members 23, 24 to the respective inner surfaces of the side sections 2, 3.

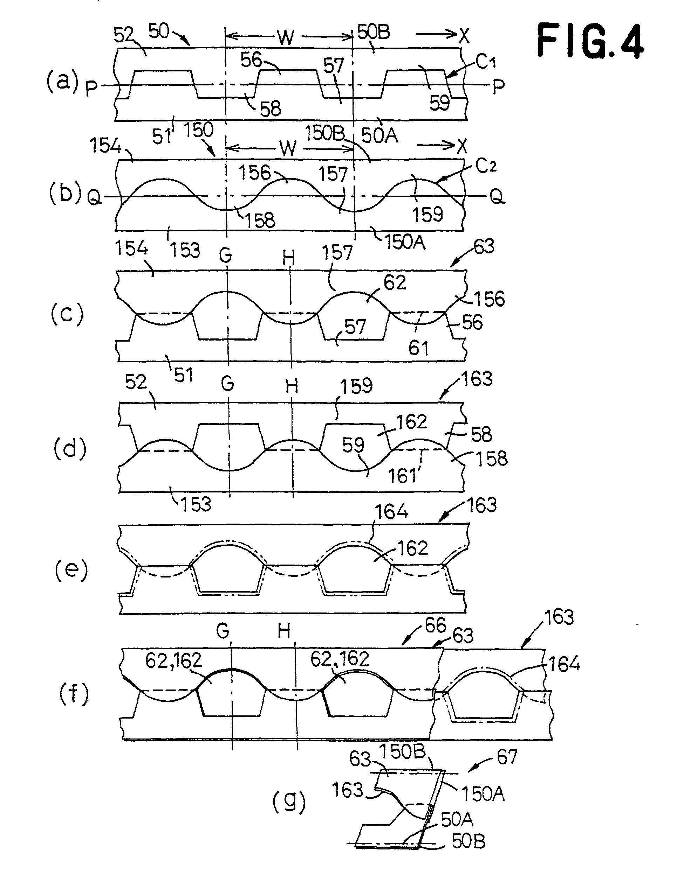

Fig. 4 is a schematic diagram illustrating the steps of the process for making the crotch member 10 according to another preferred embodiment of this invention. The process according to this embodiment produces the unit 67 comprising first and second main webs 50, 150 and destined to define the crotch member 10.

Referring to Fig. 4, on a step a), the first main web 50 is cut along a first sinusoidal wave-like curve C₁ of a wavelength W extending in a direction of a center line P-P defining the X-axis so as to divide the first main web 50 in two and thereby the first web 51 and the second web 52 are obtained.

On a step b), the second main web 150 is cut along a second sinusoidal wave-like curve C₂ also of the wavelength W extending in a direction of a center line Q-Q defining the X-axis so as to divide the second main web 150 in two and thereby third and fourth webs 153, 154 are obtained.

On a step c), the first and fourth webs 51, 154 of these webs are placed upon each other so that the crests 56 of the web 51 partially overlap the corresponding crests 156 of the web 154 and bonded to each other along the sealing line 61 to obtain the first apertured sheet 63 of which the aperture 62 is formed by each pair of troughs 57, 157 facing each other.

On a step d), the second and third webs 52, 153 are placed upon each other so that the crests 58 of the web 52 partially overlap the corresponding crests 158 of the web 153 and bonded to each other along a sealing line 161 to obtain the second apertured sheet 163 of which the aperture 162 is formed by each pair of troughs 59, 159 facing each other and repeated at the same intervals as those in the first apertured sheet 63.

On a step e), one of the first and second apertured sheets 63, 163, for example, the second apertured sheet 63 is reversed in the case illustrated.

On a step f), the first apertured sheet 63 and the reversed second apertured sheet 163 are placed upon so that the apertures 62 of the sheet 63 is aligned with the corresponding aperture 162 of the sheet 163 and bonded to each other by a sealing line 164 extending along the periphery of the second aperture 162. In this way, the laminated sheet 66 is obtained.

Finally on a step g), the laminated sheet 66 is cut along a first cutting line G (See Fig. 4(f)) extending transversely thereof so as to divide the aligned apertures 62, 162 in two and along a second cutting line H (See Fig. 4(f)) extending transversely thereof so as to divide a section extending between each pair of adjacent sets of aligned apertures 62; 162 and 62; 162 in two.

Similarly to the unit 67 of Fig. 3, the unit 67 of Fig. 4 may be provided with the sheets destined to form the left- and right-hand lateral bodies 2, 3 and the elastic members to finish the trunks 1. When the process as illustrated by Fig. 4 is adopted, the first main web 50 and the second main web 150 differing from each other in their external appearances such as color and pattern as well as in their physical properties such as elasticity and breathability may be used to obtain the unit 67 of which the front body presents properties different from those presented by the rear body. For example, a relatively inexpensive inelastic nonwoven fabric such as a spun bond nonwoven fabric may be used as stock material for the front body to reduce a material cost. On the other hand, a relatively expensive elastic nonwoven fabric made of crimped conjugated fibers or the like may be used as stock material for the rear body to improve a compatibility of the trunks with movement of the wearer's hip. It is also possible, as illustrated in Fig. 4, to cut the first main web 50 and the second main web 150 along the first and second curves C₁, C₂ of different waveforms, respectively, so that one of these two curves may improve a fitting of the trunks to the wearer's belly and the other may improve a fitness of the trunks to the wearer's back.

To exploite this invention, a nonwoven fabric, a woven fabric, a plastic film, etc. may be used as stock material for the first main web 50 and the second main web 150. While it is possible to bond these main webs 50, 150 by sewing up them, adhesion by means of hot melt adhesive or the like, or sealing by means of heat- or ultrasonic-sealing technique is preferably adopted in view of a manufacturing cost. For laminating and bonding the first - fourth webs 51, 52, 153, 154 together, the order in which these webs are placed one upon another is not critical. Furthermore, the waveforms of the curves C₁, C₂ are optional and, for example, the first and second curves C₁, C₂ may have a common waveform in the process of Fig. 4. It is also possible to carry out the step of bonding the sheets destined to form the left- and right-hand lateral bodies 2, 3 to the crotch member unit 67 before the step of cutting the laminated sheet 66 along the first and second cutting lines G, H to obtain the individual units 67. The steps of the process illustrated by Fig. 3 may be modified, for example, so that the continuous sheets intended to form the respective lateral bodies 2, 3 are bonded to the continuous laminated sheet 66 to obtain the second laminated sheet which is, in turn, cut along the cutting lines extending transversely of this second laminated sheet to obtain the individual trunks 1. The crotch member 10, i.e., the unit 67 obtained by such modified process should be understood to be within the scope of this invention.

The process according to this invention enables a relatively inexpensive nonwoven fabric to be utilized without any material loss and thereby to make the trunks-type garment in a continuous manner. Such process is particularly suitable for making disposable trunks given the top priority to the product price more or less at the cost of feeling to wear.

The process according to this invention for making the trunks-type garment allows the webs to be cut without any material loss and thereby to alleviate the product cost.

## Claims

1. A process for making a trunks-type garment having a pair of lateral bodies intended to cover a wearer's both sides and a middle body intended to cover middle and crotch regions of the wearer's trunk and bonded to said lateral bodies on belly and back sides of said wearer, said process being characterized by that said middle body is made at least through the steps of:
a. cutting a main web having first and second side sections extending in parallel to each other and continuously fed in one direction along a first sinusoidal wave-like curve extending in said one direction so as to divide said main web in two, said first curve having a given amplitude and defined by crests and troughs alternating every 1/2 wavelength, to obtain a first web having said first side section and a third side section extending substantially in parallel to said first side section so as to be contoured by said first sinusoidal wave-like curve and a second web having said second side section and a fourth side section contoured by said first sinusoidal wave-like curve from said main web;
b. bonding one of said second web and third web having a rectilinear fifth side section and a sixth side section extending substantially in parallel to said fifth side section so as to be contoured by a second sinusoidal curve having the same wavelength as said first sinusoidal curve to said first web so that the crests as well as the troughs of said one web face the corresponding crests as well as the corresponding troughs of said first web, respectively, and the corresponding crests of these two webs partially overlap in the vicinity of their summits, respectively, to obtain an apertured sheet of which each aperture is formed by a pair of said troughs facing each other;
c. placing a pair of said apertured sheets one upon another so that the apertures of these two sheets are exactly aligned with each other and bonding these two sheets along peripheries of the apertures to obtain a laminated sheet; and
d. cutting said laminated sheet along a first cutting line extending transversely of said laminated sheet so as to divide said aperture in two and along a second cutting line extending transversely of said laminated sheet so as to divide a section defined between a pair of adjacent apertures in two to obtain said middle body.

2. A process according to Claim 1, further comprising, before said step b), a step of dividing a second main web continuously fed in one direction along said second sinusoidal wave-like curve in two sections and obtaining said third web from these two sections.

3. A process according to Claim 1 or 2, wherein said first and second webs are made of a nonwoven fabric and/or a woven fabric having external appearances and/or physical attributes differing from those of a nonwoven fabric or a woven fabric used as stock material for said third web.

4. A process according to any one of Claims 1 - 3, wherein said first and second sinusoidal wave-like curves are identical in the wavelength but different in the waveform.
